# EUROPÄISCHE PATENTSCHRIFT

(11) **EP 0 748 325 B1**
(45) Veröffentlichungstag und Bekanntmachung des Hinweises auf die Patenterteilung: **03.02.1999**
(21) Anmeldenummer: 95910520.6
(22) Anmeldetag: 24.02.1995
(51) Int. Cl.: C07F 9/30, C07D 453/04, C07C 227/36, C07B 57/00

(54) **VERFAHREN ZUR HERSTELLUNG VON [L]- ODER [D]-HOMOALANIN-4-YL-(METHYL)PHOSPHINSÄURE UND DEREN SALZEN DURCH RACEMATSPALTUNG**
PROCESS FOR PREPARING [L]- OR [D]-HOMOALANIN-4-YL-(METHYL)PHOSPHINIC ACID AND ITS SALTS BY RACEMIC RESOLUTION
PROCEDE DE PREPARATION D'ACIDE HOMOALANIN-4-YL-(METHYL)PHOSPHINIQUE [L] OU [D] ET DE SES SELS PAR DECOMPOSITION RACEMIQUE

(30) Priorität: 04.03.1994 DE 4407197
(43) Veröffentlichungstag der Anmeldung: 18.12.1996
(73) Patentinhaber: Hoechst Schering AgrEvo GmbH, 13509 Berlin (DE)
(72) Erfinder: KNORR, Harald, D-60529 Frankfurt am Main (DE); SCHLEGEL, Günter, D-65835 Liederbach (DE); STARK, Herbert, D-65779 Kelkheim (DE)
(86) Internationale Anmeldenummer: EP9500682
(87) Internationale Veröffentlichungsnummer: WO9523805

(56) Entgegenhaltungen:
- EP-A- 0 057 092
- EP-A- 0 342 575
- EP-A- 0 382 113
- EP-A- 0 499 376
- CH-A- 551 945
- DE-A- 3 706 022
- CHEMICAL ABSTRACTS, vol. 68, no. 3, 15.Januar 1968 Columbus, Ohio, US; abstract no. 13342, S.YOSHIKAWA ET AL. 'The racemization of l-glutamic acid by the catalytic action of salicylaldehyde derivatives in the presence of metal ion' & KOGYO KAGAKU ZASSHI, Bd. 70, Nr. 3, 1967 Seiten 331-336,
- BULLETIN OF THE CHEMICAL SOCIETY OF JAPAN, Bd. 51, Nr. 8, 1978 TOKYO, JP;, Seiten 2366-2368, M. ANDO & S. EMOTO 'Catalytic Activities of Salicylaldehyde Derivatives VIII. Kinetic Studies of the Catalytic Racemization of Glutamic Acid at 25 C'
- BULLETIN OF THE CHEMICAL SOCIETY OF JAPAN , Bd. 42, Nr. 9, 1969 TOKYO, JP;, Seiten 2628-2631, M.ANDO & S. EMOTO 'Catalytic Activities of Salicylaldehyd Derivatives: II. Kinetic Studies of the Racemisation of Amino Acid'
- BULLETIN OF THE CHEMICAL SOCIETY OF JAPAN, Bd. 36, Nr. 6, - 1963 TOKYO, JP;, Seiten 734-738, K.TOI ET AL. 'Synthetic Resins Catalyzing the Racemization of Amino Acids. I. The Preparation of the Resins'
- CHEMICAL ABSTRACTS, vol. 91, no. 24, 10.Dezember 1979 Columbus, Ohio, US; abstract no. 193857v, I.A.YAMSKOV ET AL. 'Preparation of poly(acylamidosalicyladehyde)and the study of its ability to catalyze the racemisation of optically active amino acids' & VYSOKOMOL. SOEDIN., SER. A, Bd. 21, Nr. 8, 1979 Seiten 1838-1842,
- JOURNAL OF THE AMERICAN CHEMICAL SOCIETY , Bd. 76, - 1954 WASHINGTON, US;, Seiten 653-655, M.IKAWA & E.E.SNELL 'Benzene Analogs of Pyridoxal. The Reactions of 4-Nitrosalicylaldehyde with Amino Acids'

## Beschreibung

[DL]-Homoalanin-4-yl-(methyl)phosphinsäure (DL-Ia) bzw. deren Ammoniumsalz (DL-Ib) sind herbizid wirksame Aminosäurederivate (DE-A-27 17 440). Die Aminosäurederivate wirken in der L-Form (L-la bzw. L-lb), während die jeweilige enantiomere D-Form nahezu unwirksam ist (DE-A-2856260).

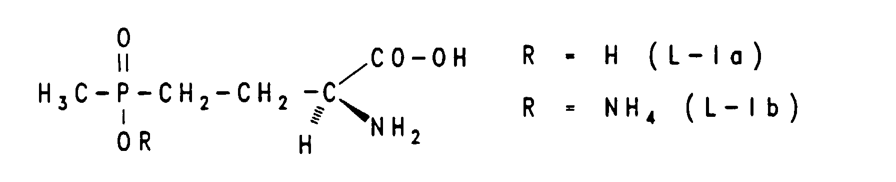

Um den reinen Wirkstoff einsetzen zu können, sind spezielle Verfahren zur Herstellung von [L]-Homoalanin-4-yl-(methyl)phosphinsäure und deren Ammoniumsalz entwickelt worden.

Nach DE-A-3 920 570 und DE-A-3 923 650 kann die L-Form durch enzymatische Transaminierung erhalten werden. Die Aufarbeitung der Transaminierungslösung ist jedoch technisch sehr aufwendig; zudem fallen große Salzmengen an.

Nach EP-A-224 880 erhält man ausgehend von [D]-Valin über mehrere Stufen, mit einer enantioselektiven Alkylierung von [R]-3-lsopropyl-2,5-dialkoxy-3,6-dihydropyrazinen als Schlüsselreaktion, ebenfalls die reine L-Form; nachteilig für die Anwendung im industrielle Maßstab sind jedoch die schwere Zugänglichkeit der heterocyclischen Zwischenprodukte und die Notwendigkeit des Einsatzes von metallorganischen Agenzien.

Außerdem gelangt man zur L-Form durch asymmetrische Hydrierung von N-substituierten 2-Amino-4-[(methyl)(hydroxy)phosphino]-butensäuren (EP-A-238954).

Ausgehend von L-Vinylglycin oder (subst.) L-4-Vinyl-1,3-oxazolidin-5-onen und Methanphosphonigsäuremonoester kann (L-la) ebenfalls hergestellt werden (EP-A-546566 bzw. EP-A-346658); die chiralen Vorprodukte sind jedoch nicht leicht zugänglich.

Weiterhin sind Verfahren unter Verwendung von Methanphosphonigsäurediestern bekannt (EP-A-508298 und EP-A-530506); die benötigte Phosphorkomponente ist jedoch in größeren Mengen nicht verfügbar, was die Realisierung dieser Verfahren in größerem Maßstab erschwert.

Eine praktikable Trennung des racemischen Gemischs von [DL-la] in die reinen Enantiomeren nach der "klassischen" Fällungsmethode unter Ausnutzung unterschiedlicher Löslichkeiten diastereomerer Salze ist bisher nicht bekannt. Für strukturell andere Aminosäuren sind einige Verfahren beschrieben, wonach Racemate mit Hilfe von chiralen Verbindungen über diastereomere Salze getrennt werden können. Besonders interessant sind dabei Verfahren, in denen die Fällung eines diastereomeren Salzes des gewünschten Enantiomers mit der Racemisierung des nicht gewünschten Enantiomers kombiniert wird.

Beispielsweise beschreibt Bull. Chim. Soc. Jap. 56 (1983) 3744-3747 die Herstellung von [D]-Phenylglycin aus [DL]-Phenylglycin mit Hilfe von [d]-Campher-10-sulfonsäure als Salzbildner in Gegenwart von Essigsäure und Salicylaldehyd als Racemisierungsmittel in 68 % Ausbeute und der optischen Reinheit von 95,9 %.

Shiraiwa et al. beschreiben in Chem. Lett. 1990, 233 f. ein Verfahren zur Herstellung von N-Methyl-[D]-2-phenylglycin aus N-Methyl-[DL]-2-phenylglycin mit [I]-Camphersulfonsäure in Butansäure ohne Zusatz von Aldehyden oder Ketonen. Dabei fällt das Salz der D-Aminosäure aus, während die L-Aminosäure racemisiert. Mit Hilfe von Triethylamin wird anschließend die D-Aminosäure aus dem diastereomeren Salz in Ausbeuten von 71-77 % freigesetzt.

US-A-4 647 692 beschreibt die Racematspaltung der Aminosäuren 4-Hydroxyphenylglycin bzw. 3,4-Dihydroxyphenylglycin durch Fällung mit (+)-3-Bromcampher-10-sulfonsäure in Gegenwart von Ketonen und organischen Säuren, wie Essigsäure. In allgemeiner Form wird diese Methode auch zur Racemattrennung von DL-la empfohlen.

Unabhängig von den obigen Fällungsmethoden, welche Racematspaltung und Racemisierung des falschen Enantiomers kombinieren, sind Veröffentlichungen bekannt, die nur Racemisierungsmethoden beschreiben:

J. Org. Chem. 48 (1983) 843-846 betrifft die Racemisierung von D-Aminosäuren in Essigsäure oder anderen organischen Carbonsäuren in Gegenwart katalytischer Mengen von aliphatischen oder aromatischen Aldehyden.

Aus US-A-3213106 ist bekannt, optisch aktive Aminosäuren in Wasser ohne Zusatz starker Basen oder Säuren bei Temperaturen von 150-250°C zu racemisieren; ferner kann man laut JP-42-13445 Aminosäuren in Wasser und in Gegenwart eines aliphatischen Aldehyds unter Metallionen-Katalyse racemisieren. Die letztgenannten Racemisierungsmethoden haben den Nachteil, daß die Aminosäuren bei den genannten Temperaturen teilweise zersetzt werden oder die Umsetzungsrate viel zu gering ist.

Eine Anwendung der weiter oben erwähnten Fällungsmethoden zur Trennung von [DL]-Homoalanin-4-yl-(methyl)phosphinsäure mit d- oder I-Camphersulfonsäure oder deren Derivaten erweist sich als nicht praktikabel. Beispielsweise gelingt die Abtrennung des diastereomeren Salzes aus [L]-Homoalanin-4-yl-(methyl)phosphinsäure und [d]-3-Brom-campher-10-sulfonsäure nicht, wie aus den Vergleichsbeispielen A) und B) (siehe Abschnitt "Vergleichsbeispiele") hervorgeht.

Es bestand deshalb die Aufgabe, ein im industriellen Maßstab durchführbares Racematspaltungsverfahren zu finden, mit dem oben beschriebene Nachteile weitgehend vermieden werden.

Gegenstand der Erfindung ist ein Verfahren zur Herstellung von [L]-Homoalanin-4-yl-(methyl)phosphinsäure (L-Säure) und deren Salzen oder von [D]-Homoalanin-4-yl-(methyl)phosphinsäure (D-Säure) und deren Salzen aus racemischer [DL]-Homoalanin-4-yl-(methyl)phosphinsäure (DL-Säure) oder deren Salzen, dadurch gekennzeichnet, daß man
a) DL-Säure oder deren Salz mit einer chiralen Base umsetzt,
b) aus einer Lösung des erhaltenen Gemischs der diastereomeren Salze aus D-Säure, L-Säure und der chiralen Base in einem wäßrigen oder wäßrig-organischen Lösungsmittel, in welchem das Salz der D-Säure bzw. der L-Säure eine höhere Löslichkeit als das Salz der L-Säure bzw. D-Säure aufweist, das Salz der L-Säure bzw. D-Säure und der chiralen Base auskristallisieren läßt (Racematspaltung) und
c) im Falle, daß die freie L-Säure bzw. D-Säure hergestellt wird, das erhaltene Salz mit einer Säure neutralisiert oder,
   im Falle, daß ein anderes Salz als das nach b) erhaltene hergestellt wird, eine Umsalzung durchführt.

Das erfindungsgemäße Verfahren zur Herstellung der [L]-Homoalanin-4-yl-(methyl)phosphinsäure und deren Salzen wird mit chiralen Basen, vorzugsweise Alkaloidbasen wie Chinin, Cinchonidin, sowie Brucin durchgeführt. Besonders vorteilhaft ist die Verwendung von Chinin.

Zur Herstellung der [D]-Homoalanin-4-yl-(methyl)phosphinsäure eignen sich die Enantiomere der genannten chiralen Basen, z.B. Chinidin und Cinchonin.

Wegen der größeren wirtschaftlichen Bedeutung der L-Säure werden im folgenden die Verfahrenswege am Beispiel der Herstellung von L-Säure beschrieben. Bei Einsatz der enantiomeren chiralen Basen lassen sich die Verfahren analog zur Herstellung der D-Säure einsetzen.

Um die Löslichkeit diastereomerer Salze der [L]-Form im Vergleich zur Löslichkeit in rein wäßrigen Lösungen herabzusetzen, können beispielsweise Lösungsmittelgemische aus Wasser und organischen Lösungsmitteln, die in dem jeweils verwendeten Mengenverhältnis mit Wasser mischbar sind, eingesetzt werden. Geeignete Mischungspartner für die wäßrig-organischen Lösungsmittelgemische sind beispielsweise organische Lösungsmittel aus der Gruppe der Alkohole, wie z.B. Methanol, Ethanol, n-Propanol, i-Propanol, n-Butanol, i-Butanol, sek.-Butanol und t-Butanol, der Ketone, wie z.B. Aceton, Methylethylketon, Methylisobutylketon und N-Methylpyrrolidon, und Kombinationen der genannten Lösungsmittel. Mit vergleichsweise geringen Mengen an Lösungsmittel, d. h. unter Verwendung hochkonzentrierter Lösungen, ist es auch möglich, Wasser als alleiniges Lösungsmittel zu verwenden.

Besonders vorteilhaft erweist sich die Verwendung von i-Propanol oder t-Butanol in Kombination mit Wasser.

Die optimale Temperatur für die Kristallisation hängt von der chiralen Base, dem Lösungsmittel, der Konzentration des Salzes, der Menge der chiralen Base und der Kristallisationsgeschwindigkeit ab. In der Regel ist es vorteilhaft, die Kristallisation bei Temperaturen von 0-100°C, vorzugsweise 0-85°C, insbesondere bei 15-75°C durchzuführen. Bevorzugt eignen sich Lösungsmittelgemische aus Wasser und Alkoholen, z.B. t-Butanol:Wasser im Verhältnis von beispielsweise 20:80 bis 90:10, vorzugsweise 50:50 bis 85:15, insbesondere 70:30 bis 85:15, oder Isopropanol:Wasser im Verhältnis von 20:80 bis 90:10, vorzugsweise 50:50 bis 90:10, insbesondere 70:30 bis 85:15. Die letztgenannten Mengenverhältnisse gelten vorzugsweise für die Durchführung der Kristallisation bei Temperaturen von 0-85°C, insbesondere 15-75°C.

Die Freisetzung der Säure (L-la) aus dem diastereomeren Salz des Kristallisats kann analog üblichen Methoden erfolgen, beispielsweise durch Neutralisation mit einer organischen oder anorganischen Säure, gegebenenfalls in einem geeigneten Lösungsmittel. Die Umsalzung in ein anderes Salz kann durch Reaktion mit einem Überschuß einer anorganischen Base, welche das gewünschte Kation enthält, erfolgen oder mit einer organischen Base (z.B. Aminbase) oder Ammoniak, wenn gegebenenfalls substituierte Ammoniumsalze von (L-la) hergestellt werden sollen. Bevorzugt ist die Herstellung des als Herbizid gut einsetzbaren Ammoniumsalzes (L-lb) durch Umsalzung mit Ammoniak. Die Reaktion mit Ammoniak läßt sich beispielsweise so durchführen, daß man das Kristallisat in einem geeigneten Lösungsmittel wie Methanol auflöst und Ammoniak einleitet oder mit einer Lösung von Ammoniak in einem Lösungsmittel, beispielsweise wieder Methanol, im Überschuß versetzt und das Ammoniumsalz (L-lb) ausfällt. Die Mutterlauge, welche die chirale Base enthält, kann dann in den nächsten Ansatz zurückgeführt werden.

In einer bevorzugten Verfahrensweise des erfindungsgemäßen Verfahrens wird das unerwünschte D-lsomer (D-la) oder dessen Salze, z.B. das Salz von (D-la) mit der chiralen Base, racemisiert und die erhaltene racemische Verbindung (DL-la) bzw. dessen Salz für die erfindungsgemäße Racematspaltung verwendet.

Für die Racemisierung von (D-la) sind prinzipiell Verfahren geeignet, mit denen auch andere Aminosäuren racemisiert werden können. Beispielsweise ist aus den bereits oben erwähnten Literaturstellen Bull. Chim. Soc. Jap. 56 (1983) 3744-3747, Chem. Lett. 1990, 233 f., J. Org. Chem. 48 (1983) 843-846 bekannt, Racemisierungen von optisch aktiven Aminosäuren durch Aldehyde in organischen Säuren zu katalysieren.

Die Racemisierung kann separat oder mit der Racemattrennung erfolgen:
a) Für die Durchführung der Racemisierung des D-lsomers nach Abtrennung des kristallisierten Salzes des L-lsomers bieten sich die vorstehend erwähnten Verfahren an (siehe auch Beispiel C in Abschnitt "Vergleichsbeispiele", weiter unten). Abgesehen von den zusätzlichen Verfahrensstufen bei separater Racemisierung haben die bekannten Verfahren aber meist weitere Nachteile, z.B. den, daß die Racemisierung in Gegenwart von Säuren erfolgen muß. Der Zusatz von organischen Säuren hat erhebliche verfahrenstechnische Nachteile, wenn man die bekannte Methode auf die Racemisierung des erfindungsgemäß in der Mutterlauge der Kristallisationsstufe anfallenden Salzes des D-lsomers und der chiralen Base anwendet. Der Zusatz von Säure bedeutet z.B. einen Wechsel des Lösungsmittels, weshalb die Racemisierungslösung nicht direkt wieder in den nächsten Kristallisationsansatz zurückgeführt werden kann, ohne die Kristallisationbedingungen zu verändern.
b) Wenn darüber hinaus die Racemisierung des D-lsomers in demselben Reaktionsansatz gleichzeitig mit der erfindungsgemäßen Kristallisation des Salzes des L-lsomers und der chiralen Base ablaufen soll, lassen sich die bekannten Racemisierungsmethoden nicht mehr anwenden oder sind industriell nicht praktikabel, wie man aus dem Vergleichsbeispiel D weiter unten erkennt. Zwar läßt sich das diastereomere Salz aus (D-la) und der chiralen Base, hier Chinin, glatt in Essigsäure in Gegenwart von Salicylaldehyd racemisieren (Vergleichsbeispiel C), doch gelingt die Kristallisation des diastereomeren Salzes von (L-la) in essigsaurem Medium nicht (Vergleichsbeispiel D).

Die bei den bekannten Racemisierungsmethoden zugesetzten organischen Säuren müssen aus den vorstehenden Gründen bei dem bevorzugten erfindungsgemäßen kombinierten Kristallisationsverfahren vermieden werden. Die Racemisierung in Gegenwart der bekannten Aldehyde gelingt ohne Zusatz von Säuren in der Regel nicht, d. h. wenn man die Racemisierung mit den Aldehyden im neutralen oder schwach basischen oder selbst schwach sauren wäßrigen Medium durchführen will.

Überraschenderweise haben unsere Versuche gezeigt, daß die Racemisierung selbst in solchen Medien gelingt, wenn man bestimmte Aldehyde verwendet. Gegenstand der Erfindung ist deshalb auch ein neuartiges Verfahren zur Racemisierung von optisch aktiven Aminosäuren (D-la bzw. L-la) und deren Salzen, vorzugsweise Aminosäuren der Formel (D-la) und deren Salzen, dadurch gekennzeichnet daß man die optisch aktiven Aminosäuren in Gegenwart von sechsgliedrigen (hetero)aromatischen Aldehyden, die eine Hydroxygruppe in 2-Position zur Aldehydgruppe und elektronenziehende Reste, wie z.B. NO₂, CN, CF₃ und SO₃H, insbesondere NO₂, in 3- oder 5-Position zur Aldehydgruppe aufweisen und gegebenenfalls weiter substituiert sind, in wäßrigem oder wäßrig-organischen Medium umsetzt.

Die erfindungsgemäße Racemisierung gelingt ohne Zusatz von anorganischen oder organischen Säuren. Bevorzugt ist die Racemisierung im neutralen oder schwach basischen oder schwach sauren Medium, beispielsweise bei pH 4-9, insbesondere bei pH 5-8.

Die Racemisierung wird in der Regel bei Temperaturen von 0-120°C, vorzugsweise 30-85°C, insbesondere 35-75°C, in Abhängigkeit von der Reaktivität des Aldehyds durchgeführt.

Bevorzugte Aldehyde für die Racemisierung sind Salicylaldehyde, die am Phenylring durch elektronenziehende Reste in 3- oder 5-Position, z.B. Nitrogruppen aktiviert sind, und gegebenenfalls weiter substituiert sind, beispielsweise 5-Nitrosalicylaldehyd oder 3,5-Dinitrosalicylaldehyd.

Es ist beispielsweise auch möglich, anstelle der aromatischen Aldehyde analoge heteroaromatische Aldehyde einzusetzen. Erwähnenswert sind hierbei Pyridinaldehyde, z.B. Pyridoxal, die je nach Substitutionsmuster auch an einen anorganischen oder organischen Träger fixiert sein können.

Als Aminosäuren kommen die optisch aktiven Aminosäuren (D- oder L-la) und deren Salze in Frage.

Die Einsatzmenge der Aldehyde kann in weiten Grenzen variiert und in Vorversuchen leicht optimiert werden. Bevorzugt werden Aldehyde im Unterschuß bezogen auf die Aminosäure oder deren Salz, insbesondere in katalytischen Mengen eingesetzt. In der Regel liegen die Mengen des jeweiligen Aldehyds im Bereich von 0,01 Mol und 0,1 Mol pro Mol eingesetzter Aminosäure bzw. deren Salz. Setzt man sehr wenig Aldehyd ein, verläuft die Umsetzung für die Praxis zu langsam. Der Einsatz von zu großen Mengen an Aldehyd kann die Weiterverarbeitung des Ansatzes beeinträchtigen und erscheint aus ökonomischer Sicht auch wenig sinnvoll.

Ein besonderer Vorteil der erfindungsgemäßen Racemisierung liegt darin, daß sie bei wesentlich niedrigeren Temperaturen durchgeführt werden kann, als zu erwarten war. Die Umsetzung gelingt nicht nur bei den in stark sauren Medien verwendeten Temperaturen von 80-150°C, sondern läßt sich bei Temperaturen unter 80°C, vorzugsweise 35-75°C, insbesondere 40-70°C durchführen. Im Gegensatz zu den genannten herkömmlichen Methoden ermöglichen diese niedrigen Temperaturen bei der Racemattrennung von (DL-la) mit chiralen Basen, die Kristallisation des Salzes von (L-la) und der chiralen Base gleichzeitig mit der Racemisierung des Salzes von (D-la) in einem Ansatz durchzuführen.

Die verschiedenen Möglichkeiten, die erfindungsgemäße Racemattrennung und die erfindungsgemäße Racemisierung der Säure (D-la) bzw. deren Salz durchzuführen, werden im folgenden erläutert.

Eine Möglichkeit besteht darin, daß man nach der Kristallisation in Stufe b) des erfindungsgemäßen Verfahrens die Mutterlauge, welche im wesentlichen das diastereomere Salz von (D-la) und Reste des diastereomeren Salzes von (L-la) enthält, in Gegenwart eines der genannten Aldehyde erwärmt, wobei die Racemisierung bei Temperaturen von 0-120°C, vorzugsweise 30-85°C, insbesondere 35-75°C, durchgeführt wird. Das Salz der racemisierten Aminosäure und der chiralen Base kann dann direkt, d. h. ohne Aufarbeitung und ohne Wechsel des Lösungsmittels, dem nächsten Kristallisationsansatz zugeführt werden.

Eine kombinierte Verfahrensweise (Alternative 1), als Batch-Verfahren oder als kontinuierliches Verfahren zur Herstellung des Ammoniumsalzes (L-lb) ausgehend von dem Ammoniumsalz (DL-Ib), ist beispielsweise dadurch gekennzeichnet, daß man
(1) in einer Lösungsmittelmischung von Wasser und einem organischen Lösungsmittel, welches [DL]-Homoalanin-4-yl-(methyl)phosphinsäureammoniumsalz anlöst, das [DL]-Homoalanin-4-yl-(methyl)phosphinsäureammoniumsalz mit einer chiralen Base umsetzt und das freigesetzte Ammoniak entfernt, danach
(2) bei Temperaturen von 0-85°C in einer Lösungsmittelmischung von Wasser und einem organischen Lösungsmittel das diastereomere Salz aus [L]-Homoalanin-4-yl-(methyl)phosphinsäure und der chiralen Base auskristallisieren läßt und isoliert, z.B. durch Absaugen, und anschließend
(3) die Mutterlauge des Kristallisats, die im wesentlichen das andere diastereomere Salz der [D]-Aminosäure und Reste des diastereomeren Salzes der [L]-Aminosäure enthält, in Gegenwart eines (hetero)aromatischen Aldehyds bei Temperaturen von 20-120°C erwärmt und nach der Racemisierung die erhaltene Lösung dem nächsten Kristallisationsansatz (2) zuführt und
(4) das diastereomere Salz aus [L]-Homoalanin-4-yl-(methyl)phosphinsäure und der chiralen Base aus Stufe (2) in der Mischung aus Wasser und einem organischen Lösungsmittel bzw. in dem organischen Lösungsmittel selbst mit Ammoniak umsetzt, wobei [L]-Homoalanin-4-yl(methyl)phosphinsäure-ammoniumsalz (L-lb) ausfällt, das ausgefallene Ammoniumsalz (L-lb) isoliert, z.B. durch Absaugen, und die Mutterlauge, welche im wesentlichen die chirale Base enthält, in Stufe (1) des nächsten Ansatzes zurückführt.

Für die Optimierung des kombinierten Verfahrens ist es bedeutsam, die Temperaturen an den jeweiligen Verfahrensschritt anzupassen. In Stufe (1) sind Temperaturen von 20-100°C vorteilhaft, während der Schritt gemäß Stufe (2) günstig bei 0-85°C, vorzugsweise bei 15-75°C durchgeführt wird. Die Temperatur in Stufe (3) sollte auf die Reaktivität des Aldehyds abgestimmt werden. Das Verfahren gemäß Stufe (4) läßt sich bevorzugt bei Temperaturen von 0-60°C durchführen.

In einer weiteren Möglichkeit, die besonders bevorzugt ist, wird die Racemisierung in derselben Stufe durchgeführt wie die Kristallisation des diastereomeren Salzes von (L-la). Die Bedingungen für die Kristallisation hinsichtlich Lösungsmittel und Temperatur stimmen dann zwangsläufig mit denen für die Racemisierung überein; dies schränkt die Wahl der Racemisierungsverfahren und im Falle der erwähnten Racemisierung mit Aldehyden, die Wahl der möglichen Aldehyde ein. Wie bereits oben erwähnt, läßt sich dieses kombinierte Verfahren nicht mit den herkömmlichen Verfahren in Gegenwart von Säuren und Aldehyden praktikabel durchführen, sondern unter Anwendung des genannten erfinderischen Verfahrens zur Racemisierung mit speziellen Aldehyden ohne Zusatz von Säuren.

Die erfinderische Variante des kombinierten Verfahrens ist dadurch gekennzeichnet, daß man ein Gemisch der diastereomeren Salze aus D-Säure ozw. L-Säure und der chiralen Base, gelöst in einem wäßrigen oder wäßrigorganischen Lösungsmittel, in welchem das Salz der D-Säure eine höhere Löslichkeit als das Salz der L-Säure aufweist, bei Temperaturen von 0-85°C, vorzugsweise 30-85°C in Gegenwart eines Aldehyds umsetzt, wobei die Temperatur so niedrig eingestellt ist, daß zugleich das Salz der L-Säure und der chiralen Base auskristallisiert.

Mit dem bevorzugten kombinierten Verfahren (Racemattrennung und Racemisierung) ist es prinzipiell möglich (DL-la) zu 100 % in (L-la) zu überführen. Für das bevorzugte Verfahren sind die obengenannten chiralen Basen und die sechsgliedrigen (hetero)aromatischen Aldehyde, die eine Hydroxygruppe in 2-Position zur Aldehydgruppe und elektronenziehende Reste in 3- oder 5-Position zur Aldehydgruppe aufweisen, insbesondere die bevorzugt erwähnten Basen und Aldehyde geeignet.

Die bevorzugte kombinierte Verfahrensweise (Alternative 2), als Batch-Verfahren oder als kontinuierliches Verfahren zur Herstellung des Ammoniumsalzes (L-lb) ausgehend von dem Ammoniumsalz (DL-Ib), ist beispielsweise dadurch gekennzeichnet, daß man
(1') in einer Lösungsmittelmischung von Wasser und einem organischen Lösungsmittel, welches [DL]-Homoalanin-4-yl-(methyl)phosphinsäureammoniumsalz anlöst, das [DL]-Homoalanin-4-yl-(methyl)phosphinsäureammoniumsalz mit einer chiralen Base umsetzt und Ammoniak entfernt, danach
(2') mit einem aromatischen Aldehyd bei Temperaturen von 0-85°C, vorzugsweise 30-85°C in Gegenwart einer Lösungsmittelmischung von Wasser und einem organischen Lösungsmittel umsetzt und zugleich das diastereomere Salz aus [L]-Homoalanin-4-yl-(methyl)phosphinsäure und der chiralen Base auskristallisieren läßt, das Kristallisat isoliert, z.B. durch Absaugen, und die Mutterlauge der Stufe (2') des nächsten Ansatzes zufügt und
(3') das diastereomere Salz aus [L]-Homoalanin-4-yl-(methyl)phosphinsäure und der chiralen Base aus Stufe (2') in der Mischung aus Wasser und einem organischen Lösungsmittel bzw. in dem organischen Lösungsmittel selbst mit Ammoniak umsetzt, dabei das ausgefallene [L]-Homoalanin-4-yl-(methyl)phosphinsäure-ammoniumsalz absaugt und die Mutterlauge, welche im wesentlichen die chirale Base enthält, in Stufe (1') des nächsten Ansatzes zurückführt.

Für das Gelingen des Verfahrens ist es wichtig die Temperaturen an den jeweiligen Verfahrensschritt anzupassen. Die Verfahrensschritte (1') und (3') entsprechen weitgehend den Verfahrensschritten (1) und (4) aus dem oben bereits erwähnten kombinierten Verfahren (Alternative 1). Für den Verfahrensschritt gemäß Stufe (1') ist es vorteilhaft bei Temperaturen von 20-100°C zu arbeiten, während der Schritt gemäß Stufe (2') günstig bei der Temperatur durchgeführt wird, bei der das diastereomere Salz von (L-Ia) auskristallisiert aber die Racemisierung des nicht gewünschten (D-la) noch ausreichend schnell abläuft.
Vorteilhaft wird Stufe (3') bei Temperaturen von 0-60°C durchgeführt.

Ein schematischer Vergleich der Alternativen 1 und 2 zeigt am Beispiel der Racemattrennung von (L-lb) die Einsparung einer Verfahrensstufe bei der Alternative 2 (siehe Tabelle 1):

**Tabelle 1**

| Nr. | Alternative 1 | Alternative 2 |
|---|---|---|
| (1) | (DL-lb) + chirale Base | (DL-lb) + chirale Base |
| (2) | Entfernen von NH₃, ggf. Lösungsmittel wechseln | Entfernen von NH₃, ggf. Lösungsmittel wechseln |
| (3) | Kristallisation (Racematspaltung) | Kristallisation (Racematspaltung) und Umsetzung mit Aldehyd |
| (4) | Filtration | Filtration Mutterlauge in (3) zurück |
| (5) | Kristallisat auflösen und mit NH₃ umsetzen | Kristallisat auflösen und mit NH₃ umsetzen |
| (6) | Produkt (L-Ib) abfiltrieren, Mutterlauge in (1) zurück | Produkt (L-lb) abfiltrieren, Mutterlauge in (1) zurück |
| (7) | Mutterlauge aus (4) mit Aldehyd erhitzen und in (3) zurück | |
| Zu Tabelle 1: (Nr.) = Nummer der Verfahrensoperation | | |

Es ist möglich die einzelnen Verfahrensschritte batchweise oder auch kontinuierlich durchzuführen. Die dabei anfallenden Mutterlaugen werden vorzugsweise in den Gesamtprozeß zurückgeführt, um Ausbeuteverluste klein zu halten.

Als Lösungsmittel für die oben beschriebenen Verfahrensstufen eignen sich die für die Kristallisationsstufe bereits erwähnten Lösungsmittel. Eine vorteilhafte Vorgehensweise besteht darin, daß man in allen Verfahrensschritten das gleiche Lösungsmittelsystem verwendet. Es ist jedoch manchmal auch sinnvoll, die Eigenschaften des Lösungsmittelsystems einfach so zu variieren, daß man dem Lösungsmittel aus der vorherigen Stufe ein anderes Lösungsmittel zusetzt.

### Beispiele

In den nachfolgenden Beispielen beziehen sich Mengen- und Prozentangaben auf das Gewicht, sofern nichts anderes angegeben ist. Die Bezeichnungen "L-Salz", "D-Salz" und "D,L-Salz" bedeuten das Salz aus (L-la), (D-la) bzw. (D,L-la) und der chiralen Base.

### Beispiel 1

1.1 39,6 g 99,8 %iges [DL]-Homoalanin-4-yl-(methyl)phosphinsäureammoniumsalz (0,2 Mol) und 65,5 g (-)Chinin (99 %ig) (0.2 Mol) werden in 210,8 g Wasser zum Rückfluß erhitzt. Anschließend werden 22,6 g wäßriges Ammoniak durch Anlegen von Unterdruck bei 100 mbar entfernt. Man fügt bei 70°C 766,4 g tert.-Butanol und dann 3,38 g 5-Nitrosalicylaldehyd (0,02 Mol) hinzu und impft die klare Lösung bei 50°C mit [L]-Homoalanin-4-yl-(methyl)phosphinsäure/Chininsalz an. Ab 48°C fällt langsam das diastereomere L-Salz aus. Man läßt innerhalb von 6 Stunden auf Raumtemperatur kommen, saugt ab, wäscht mit wenig tert.-Butanol/Wasser (80:20) und trocknet im Vakuum bei 60°C. Dabei erhält man 41,0 g [L]-Homoalanin-4-yl-(methyl)phosphinsäure/Chininsalz mit einer Reinheit von L-Salz:D-Salz von 98,7:1,3.
1.2 Die Mutterlauge aus Beispiel 1.1 wird 9 Stunden auf Rückfluß gehalten (Probemessung L:D = 50,6:49,4) und zu einem weiteren 0,2 Mol-Ansatz analog Beispiel 1.1 bei 70°C hinzugefügt (Einsatzmenge ca. 0,319 Mol [DL]-Salz). Man läßt auskristallisieren und erhält 97,3 g [L]-Homoalanin-4-yl-(methyl)phosphinsäure/Chininsalz mit einer Reinheit von L-Salz:D-Salz von 99,5:0,5 (entsprechend 60 % d. Th.). Die Mutterlauge kann wiederum einem weiteren Ansatz zugefügt werden. Das Kristallisat wird in 97,3 g Methanol aufgenommen und mit 27,8 g methanolischem Ammoniak (17,7 %ig) (0,29 Mol) versetzt; anschließend wird das gebildete Kristallisat filtriert. Man erhält 36,2 g [L]-Homoalanin-4-yl-(methyl)phosphinsäure-ammoniumsalz mit einer optischen Reinheit von L:D = 99,5:0,5. Das entspricht einer isolierten Ausbeute von 57,0 % d.Th. bezogen auf 0,319 Mol DL-Salz. Die Mutterlauge dieses Kristallisates, welche im wesentlichen das (-)Chinin enthält, wird einem weiteren Ansatz zugeführt.

### Beispiel 2

39,6 g 99,8 %iges [DL]-Homoalanin-4-yl-(methyl)phosphinsäure-ammoniumsalz (0,2 Mol) und 65,5 g (-)Chinin (99 %ig) (0,2 Mol) werden in 210,8 g Wasser erhitzt; anschließend werden 24,0 g wäßriges Ammoniak durch Anlegen von Unterdruck bei 100 mbar entfernt. Man fügt bei 70°C 766,4 g tert.-Butanol und dann 4,3 g 3,5-Dinitrosalicylaldehyd (0,02 Mol) hinzu, kühlt auf 50°C ab und impft die klare Lösung mit [L]-Homoalanin-4-yl-(methyl)phosphinsäure/Chininsalz an. Man rührt 9 bis 10 Stunden, wobei langsam L-Salz ausfällt. Man läßt innerhalb von 6 Stunden auf Raumtemperatur kommen, saugt ab, wäscht mit tert.-Butanol/Wasser (80:20) und trocknet im Vakuum bei 60°C. Dabei erhält man 86,5 g [L]-Homoalanin-4-yl-(methyl)phosphinsäure/Chininsalz mit einer Reinheit von L-Salz:D-Salz von 99,5:0,5. Das entspricht einer Ausbeute von 85,1 % d. Th. Die Mutterlauge wird zu einem weiteren Ansatz bei 70°C hinzugefügt. Das Kristallisat wird in 86,5 g Methanol aufgenommen, mit 24,7 g methanolischem Ammoniak (17,7 %ig) (0,258 Mol) versetzt und das gebildete Kristallisat filtriert. Man erhält 32,2 g [L]-Homoalanin-4-yl-(methyl)phosphinsäure-ammoniumsalz mit einer optischen Reinheit von L:D = 99,0:1,0. Das entspricht einer isolierten Ausbeute von 80,5 % d. Th. Die Mutterlauge, welche im wesentlichen das (-)Chinin erhält, wird einem weiteren Ansatz zugeführt.

### Beispiel 3

3,5 g [DL]-Homoalanin-4-yl-(methyl)phospninsäure-ammoniumsalz (0,019 Mol) und 6,2 g (-)Chinin (0,019 Mol) werden in 18,2 g Wasser bei 50°C gelöst und mit 27,4 g heißem Aceton versetzt. Man erhält bei 50°C eine klare Lösung. Man läßt langsam abkühlen, impft unterdessen die klare Lösung mit [L]-Homoalanin-4-yl-(methyl)phosphinsäure/Chininsalz an und läßt auskristallisieren. Bei 20°C wird abgesaugt, mit wenig Aceton gewaschen und der Filterkuchen bei 60°C im Vakuum getrocknet. Man erhält 4,0 g [L]-Homoalanin-4-yl-(methyl)phosphinsäure/Chininsalz, das L-Aminosäureanteil und D-Aminosäureanteil im Enantiomerenverhältnis von 99,8:0,2 enthält. Das entspricht einer Ausbeute von 83,3 % d. Th., bezogen auf den Einsatz an L-Form, und 41,7 % d. Th., bezogen auf eingesetztes D,L-Gemisch.

### Beispiel 4

3,5 g [DL]-Homoalanin-4-yl-(methyl)phosphinsäure-ammoniumsalz (0,019 Mol) und 6,2 g (-)Chinin (0,019 Mol) werden in 18,2 g Wasser bei 50°C gelöst und mit 103,1 g heißem Isopropanol versetzt. Man erhält bei 50°C eine klare Lösung. Man läßt langsam abkühlen, impft unterdessen die klare Lösung mit [L]-Homoalanin-4-yl-(methyl)phosphinsäure/Chininsalz an und läßt auskristallisieren. Bei 20°C wird abgesaugt, mit wenig Aceton gewaschen und der Filterkuchen bei 60°C im Vakuum getrocknet. Man erhält 4,2 g [L]-Homoalanin-4-yl-(methyl)phosphinsäure/Chininsalz, das L-Aminosäureanteil und D-Aminosäureanteil im Enantiomerenverhältnis von 99,8:0,2 enthält. Das entspricht einer Ausbeute von 86,3 % d. Th., bezogen auf den Einsatz an L-Form, und 43,2 % d. Th., bezogen auf eingesetztes D,L-Gemisch.

### Beispiel 5

1,1 g [D]-Homoalanin-4-yl-(methyl)phosphinsäure (D:L = 99,5:0,5) (0,006 Mol), 2,0 g Chinin (0,006 Mol) und 0,13 g 3,5 Dinitrosalicylaldehyd (0,0006 Mol) werden in 5,2 g Wasser und 23,0 g tert.-Butanol gelöst und 23 Stunden bei 40°C gerührt. Man erhält [DL]-Homoalanin-4-yl-(methyl)phosphinsäure/Chininsalz, das D-Aminosäureanteil und L-Aminosäureanteii im Enantiomerenvernältnis von 50,2:49,8 enthält.

### Beispiel 6

3,44 g [DL]-Homoalanin-4-yl-(methyl)phosphinsäure und 5,6 g Cinchonin (0,019 Mol) werden in 27 ml Wasser bei 50°C gelöst und mit 243 g tert.-Butanol heiß versetzt. Die Lösung wird mit [D]-Homoalanin-4-yl-(methyl)phosphinsäure angeimpft und langsam auf Raumtemperatur abgekühlt. Man erhält 4,5 g des [D]-Homoalanin-4-yl-(methyl)phosphinsäure/Cinchonin-Salzes mit einer Enantiomerenreinheit von D:L = 96,7:3,3. Das entspricht einer isolierten Ausbeute von 96,2 % d.Th..

### Vergleichsbeispiele

A) 2,7 g [DL]-Homoalanin-4-yl-(methyl)phosphinsäure (0,015 Mol), 5,0 g (+)-3-Bromcampher-8-sulfonsäure-ammoniumsalz (0,015 Mol) in 10 g entmineralisiertem Wasser werden bei 75°C mit 90 g tert.-Butanol so versetzt, daß die Temperatur auf 75°C gehalten wird. Man erhitzt 1 Stunde bei Rückfluß und läßt dann langsam auf Raumtemperatur kommen. Ausgefallene Kristalle werden abgesaugt, gewaschen und im Vakuum bei 50°C getrocknet. Man erhält 4,8 g des Homoalanin-4-yl-(methyl)phosphinsäure/(+)-3-Bromcampher-8-sulfonsäure-Salzes mit einem Diastereomerengehalt von L:D = 50:50.
B) 3,0 g [DL]-Homoalanin-4-yl-(methyl)phosphinsäure-ammoniumsalz (0,015 Mol), 5,0 g (+)-3-Bromcampher-8-sulfonsäure-ammoniumsalz (0,015 Mol) in 10 g entmineralisiertem Wasser werden bei 75°C gelöst und bei dieser Temperatur mit 135 g tert.-Butanol versetzt. Man fügt 1,53 g H₂SO₄ (96 %ig) (0,015 Mol) hinzu und läßt langsam abkühlen. Es werden 3,5 g Homoalanin-4-yl-(methyl)phosphinsäure/(+)-3-Bromcampher-8-sulfonsäuresalz mit einem Diastereomerengehalt von L:D = 50,1:49,9 erhalten.
C) 2,9 g [D]-Homoalanin-4-yl-(methyl)phosphinsäure/Chininsalz mit der Reinheit D-Salz:L-Salz von 99,8:0,2 (0,0057 Mol), 0,07 g Salicylaldehyd, 6,2 g Essigsäure und 0,02 g Wasser werden 8 Stunden bei 50°C gerührt. Die so erhaltene Lösung enthält das racemische Salz in einem Diasteromerenverhältnis von L-Salz:D-Salz = 49,8:50,2.
D) 6,2 g Chinin, 3,44 g [DL]-Homoalanin-4-yl-(methyl)phosphinsäure in 20 ml Essigsäure und 80 ml Methylisobutylketon werden erhitzt und langsam auf Raumtemperatur abgekühlt. Dabei kristallisieren 3,1 g [DL]-Salz vom Diastereomerenverhältnis von L:D = 49,6:50,4.

## Patentansprüche

1. Verfahren zur Herstellung von [L]-Homoalanin-4-yl-(methyl)phosphinsäure (L-Säure) und deren Salzen 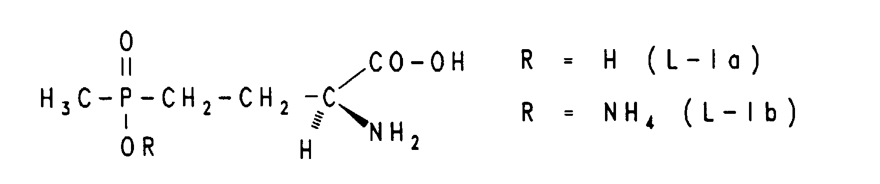 oder von [D]-Homoalanin-4-yl-(methyl)phosphinsäure (D-Säure) und deren Salzen
aus racemischer [DL]-Homoalanin-4-yl-(methyl)phosphinsäure (DL-Säure) oder deren Salzen durch Racematspaltung, dadurch gekennzeichnet, daß man
a) DL-Säure oder deren Salz mit einer chiralen Base umsetzt,
b) aus einer Lösung des erhaltenen Gemischs der diastereomeren Salze aus D-Säure, L-Säure und der chiralen Base in einem wäßrigen oder wäßrig-organischen Lösungsmittel, in welchem das Salz der D-Säure bzw. L-Säure eine höhere Löslichkeit als das Salz der L-Säure bzw. D-Säure aufweist, das Salz der L-Säure bzw. D-Säure und der chiralen Base auskristallisieren läßt (Racematspaltung) und
c) im Falle, daß die freie L-Säure bzw. D-Säure hergestellt wird, das erhaltene Salz mit einer Säure neutralisiert oder,
im Falle, daß ein anderes Salz als das nach b) erhaltene hergestellt wird, eine Umsalzung durchführt.

2. Verfahren nach Anspruch 1, dadurch gekennzeichnet, daß man L-Säure oder deren Salze hergestellt.

3. Verfahren nach Anspruch 2, dadurch gekennzeichnet, daß man als chirale Base Chinin verwendet.

4. Verfahren nach Anspruch 2 oder 3, dadurch gekennzeichnet, daß man als Lösungsmittel Lösungsmittelgemische aus Wasser und organischen Lösungsmitteln aus der Gruppe der Alkohole und Ketone einsetzt.

5. Verfahren nach Anspruch 4, dadurch gekennzeichnet, daß man ein Lösungsmittelgemisch aus Wasser und Isopropanol oder tert.-Butanol einsetzt.

6. Verfahren nach einem der Ansprüche 2-5, dadurch gekennzeichnet, daß die Stufe b) bei Temperaturen von 0-100°C durchgeführt wird.

7. Verfahren nach einem der Ansprüche 2-6, dadurch gekennzeichnet, daß das D-lsomer (D-la) oder dessen Salze racemisiert und die erhaltene racemische Verbindung (DL-la) für die Racematspaltung verwendet wird.

8. Verfahren nach Anspruch 7, dadurch gekennzeichnet, daß man das D-Isomer (D-la) in Gegenwart von sechsgliedrigen (hetero)aromatischen Aldehyden, die eine Hydroxygruppe in 2-Position zur Aldehydgruppe und elektronenziehende Reste in 3- oder 5-Position zur Aldehydgruppe aufweisen und gegebenenfalls weiter substituiert sind, in wäßrigem oder wäßrig-organischen Medium ohne Zusatz von organischen Säuren umsetzt.

9. Verfahren nach Anspruch 8, dadurch gekennzeichnet, daß man 5-Nitrosalicylaldehyd oder 3,5-Dinitrosalicylaldehyd einsetzt.

10. Verfahren nach einem der Ansprüche 7-9, dadurch gekennzeichnet, daß die Racemisierung bei Temperaturen von 0-120°C durchgeführt wird.

11. Verfahren nach einem der Ansprüche 8-10, dadurch gekennzeichnet, daß man ein Gemisch der diastereomeren Salze aus D-Säure bzw. L-Säure und der chiralen Base, gelöst in einem wäßrigen oder wäßrig-organischen Lösungsmittel, in welchem das Salz der D-Säure eine höhere Löslichkeit als das Salz der L-Säure aufweist, bei Temperaturen von 0-85°C in Gegenwart des Aldehyds umsetzt, wobei die Temperatur so niedrig eingestellt ist, daß zugleich das Salz der L-Säure und der chiralen Base auskristallisiert.

12. Salze von [L]- oder [D]-Homoalanin-4-yl-(methyl)phosphinsäure und chiralen Alkaloidbasen.

13. Salz von [L]-Homoalanin-4-yl-(methyl)phosphinsäure (L-Säure) und Chinin.

## Claims

1. A process for preparing [L]-homoalanin-4-yl(methyl)phosphinic acid (L acid) and salts thereof 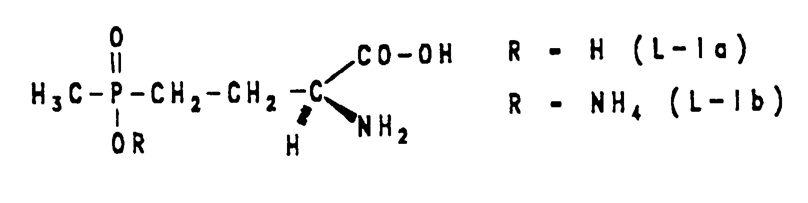 or [D]-homoalanin-4-yl(methyl)phosphinic acid (D acid) and salts thereof
from racemic [DL]-homoalanin-4-yl(methyl)phosphinic acid (DL acid) or salts thereof by racemate resolution, which comprises
a) reacting DL acid or salt thereof with a chiral base,
b) allowing the salt of the L acid or D acid and of the chiral base to crystallize out of a solution of the resulting mixture of the diastereomeric salts of D acid, L acid and the chiral base in an aqueous or aqueous-organic solvent in which the salt of the D acid or L acid has a higher solubility than the salt of the L acid or D acid, respectively (racemate resolution) and
c) in the case where the free L acid or D acid is prepared, neutralizing the resulting salt with an acid, or in the case where a salt other than that obtained according to b) is prepared, carrying out a metathesis.

2. The process as claimed in claim 1, wherein L acid or salts thereof are prepared.

3. The process as claimed in claim 2, wherein quinine is used as chiral base.

4. The process as claimed in claim 2 or 3, wherein solvent mixtures of water and organic solvents from the group of alcohols and ketones are used as solvents.

5. The process as claimed in claim 4, wherein a solvent mixture of water and isopropanol or tert-butanol is used.

6. The process as claimed in any of claims 2-5, wherein stage b) is carried out at temperatures of 0-100°C.

7. The process as claimed in any of claims 2-6, wherein the D isomer (D-Ia) or salts thereof are racemized, and the resulting racemic compound (DL-Ia) is used for the racemate resolution.

8. The process as claimed in claim 7, wherein the D isomer (D-Ia) is reacted in the presence of six-membered (hetero)aromatic aldehydes which have a hydroxyl group in position 2 with respect to the aldehyde group and electron-attracting radicals in position 3 or 5 with respect to the aldehyde group and are further substituted where appropriate, in aqueous or aqueous-organic medium without addition of organic acids.

9. The process as claimed in claim 8, wherein 5-nitrosalicylaldehyde or 3,5-dinitrosalicylaldehyde is used.

10. The process as claimed in any of claims 7-9, wherein the racemization is carried out at temperatures of 0-120°C.

11. The process as claimed in any of claims 8-10, wherein a mixture of the diastereomeric salts of D acid and L acid and of the chiral base, dissolved in an aqueous or aqueous-organic solvent in which the salt of the D acid has a higher solubility than the salt of the L acid, is reacted at temperatures of 0-85°C in the presence of the aldehyde, with the temperature being set sufficiently low for the salt of the L acid and of the chiral base to crystallize out at the same time.

12. A salt of [L]- or [D]-homoalanin-4-yl(methyl)phosphinic acid and chiral alkaloid bases.

13. A salt of [L]-homoalanin-4-yl(methyl)phosphinic acid (L acid) and quinine.

## Revendications

1. Procédé de préparation d'acide [L]-homoalanine-4-yl-(méthyl)phosphinique (acide L) ou de ses sels 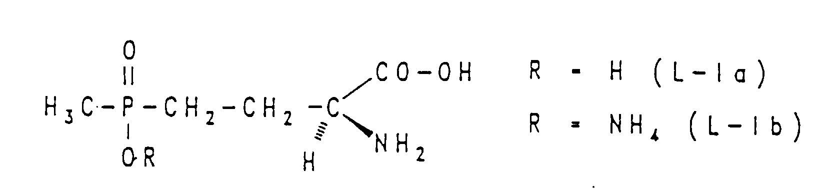 ou d'acide [D]-homoalanine-4-yl-(méthyl)-phosphinique (acide D) ou de ses sels
à partir de l'acide racémique [DL]-homoalanin-4-yl-(méthyl)-phosphinique (acide DL) ou de ses sels par résolution du racémisation, caractérisé en ce que
a) l'on met à réagir l'acide DL ou un sel de celui-ci avec une base chirale,
b) l'on fait cristalliser le sel de l'acide L, respectivement de l'acide D, et de la base chirale dans une solution du mélange obtenu des sels diastéréoisomères constitué de l'acide D, respectivement de l'acide L, et de la base chirale dans un solvant aqueux ou aqueux-organique, dans lequel le sel de l'acide D, respectivement de l'acide L, présente une plus grande solubilité que le sel de l'acide L, respectivement de l'acide D (résolution du racémique) et
c) dans le cas, où l'on prépare l'acide L libre, respectivement l'acide D, on neutralise le sel obtenu avec un acide ou,
dans le cas, où l'on prépare un autre sel que celui obtenu selon b), on réalise une conversion de sel.

2. Procédé selon la revendication 1, caractérisé en ce qu'on prépare le L-acide ou ses sels.

3. Procédé selon la revendication 2, caractérisé en ce qu'on utilise la quinine comme base chirale.

4. Procédé selon la revendication 2 ou 3, caractérisé en ce qu'on utilise comme solvant des mélanges de solvants composés d'eau et de solvants organiques choisis parmi les alcools et les cétones.

5. Procédé selon la revendication 4, caractérisé en ce qu'on utilise un mélange de solvants constitué d'eau et d'isopropanol ou de tert.-butanol.

6. Procédé selon l'une des revendications 2 - 5, caractérisé en ce qu'on réalise l'étape b) à des températures de 0 - 100°C.

7. Procédé selon l'une des revendications 2 - 6, caractérisé en ce qu'on racémise l'isomère D (D-Ia) ou ses sels et en ce qu'on utilise le composé racémique obtenu (DL-la) pour la résolution du racémique.

8. Procédé selon la revendication 7, caractérisé en ce que l'on met à réagir l'isomère D (D-Ia) en présence d'aldéhydes (hétéro)aromatiques à six chaînons, qui présentent un groupe hydroxy en position 2 par rapport au groupe aldéhyde et des groupes électrophiles en position 3 ou 5 par rapport au groupe aldéhyde et sont éventuellement encore substitués, en milieu aqueux ou aqueux-organique sans addition d'acides organiques.

9. Procédé selon la revendication 8, caractérisé en ce que l'on utilise le 5-nitrosalicylaldéhyde ou le 3,5-dinitrosalicylaldéhyde.

10. Procédé selon l'une des revendications 7 - 9, caractérisé en ce qu'on réalise la racémisation à des températures de 0 - 120°C.

11. Procédé selon l'une des revendications 8 - 10, caractérisé en ce l'on dissout un mélange des sels diastéréoisomères du D-acide et du L-acide et de la base chirale dans un solvant aqueux ou aqueux-organique, dans lequel le sel du D-acide présente une solubilité plus élevée que le sel du L-acide, à des températures de 0 - 85°C, en présence de l'aldéhyde, dans lequel la température est ajustée à une valeur si basse que le sel du L-acide et de la base chirale cristallise en même temps.

12. Sels de l'acide [L]- ou [D]-homoalanin-4-yl-(méthyl)-phosphinique et de bases alcaloïdes chirales.

13. Sel de l'acide [L]-homoalanin-4-yl-(méthyl)-phosphinique (L-acide) et de la quinine.
